# EUROPÄISCHE PATENTSCHRIFT

(11) **EP 0 533 723 B1**
(45) Veröffentlichungstag und Bekanntmachung des Hinweises auf die Patenterteilung: **15.11.1995**
(21) Anmeldenummer: 91910557.7
(22) Anmeldetag: 06.06.1991
(51) Int. Cl.: C07H 15/04

(54) **VERFAHREN ZUR AUFHELLUNG VERFÄRBTER OBERFLÄCHENAKTIVER ALKYLGLYKOSIDE UND AUFBEREITUNG DES GEBLEICHTEN GUTES**
PROCESS FOR BRIGHTENING DISCOLOURED SURFACE-ACTIVE ALKYL GLYCOSIDES AND FOR PROCESSING THE BLEACHED MATERIAL
PROCEDE POUR ECLAIRCIR DES ALKYLGLYCOSIDES TENSIOACTIFS DECOLORES ET POUR TRAITER LE PRODUIT BLANCHI

(30) Priorität: 15.06.1990 DE 4019175
(43) Veröffentlichungstag der Anmeldung: 31.03.1993
(73) Patentinhaber: Henkel Kommanditgesellschaft auf Aktien, 40191 Düsseldorf (DE)
(72) Erfinder: WÜST, Willi, D-4030 Ratingen (DE); ESKUCHEN, Rainer, D-4000 Düsseldorf 13 (DE); SCHULZ, Paul, D-5600 Wuppertal (DE); BAUER, Volker, D-4000 Düsseldorf (DE); CARDUCK, Franz-Josef, D-5657 Haan (DE); ESSER, Herbert, D-5210 Troisdorf (DE); ZEISE, Christiane, D-4052 Korschenbroich 2 (DE); WEUTHEN, Manfred, D-5650 Solingen 11 (DE); PENNINGER, Josef, D-4010 Hilden (DE)
(86) Internationale Anmeldenummer: EP9101047
(87) Internationale Veröffentlichungsnummer: WO9119723

(56) Entgegenhaltungen:
- EP-A- 0 165 721
- EP-A- 0 306 652
- EP-A- 0 365 831

## Beschreibung

Oberflächenaktive Alkylglykoside sind die bekannten Reaktionsprodukte aus Zuckern und längerkettigen Alkoholen, insbesondere aliphatischen primären Alkoholen mit vorzugsweise 8 bis 22 Kohlenstoffatomen. Als Zuckerkomponente kommen dabei die als Glykosen bezeichneten Aldosen bzw. auch Ketosen im breiten Wortsinne in Betracht, von denen beispielhaft benannt seien die Glucose, Fructose, Manose, Galactose, Talose, Gulose, Allose, Altrose, Idose, Arabinose, Xylose, Lyxose und Ribose. Wegen ihrer erhöhten Reaktionsfähigkeit werden in der Alkylglykosid-Synthese insbesondere Aldosen verwendet. Hier kommt der Glucose wegen ihrer leichten Zugänglichkeit und Verfügbarkeit in technischen Mengen besondere Bedeutung zu. Die in der Praxis bevorzugten Verbindungen der hier betroffenen Art sind dementsprechend oberflächenaktive Alkylglukoside. Ihr Alkylrest leitet sich vorzugsweise von primären aliphatischen Alkoholen synthetischen, insbesondere aber von solchen natürlichen Ursprungs ab. Der Fettalkoholrest liegt dabei in acetalischer Bindung an einem einkernigen Glucoserest und/oder an Oligo- bzw. Polysaccharidreste gebunden vor. Für die Zwecke der Erfindung werden diese Begriffe als untereinander gleichbedeutend angesehen.

Oberflächenaktive Alkylglykoside werden entweder auf dem Wege der sogenannten Direktsynthese aus den längerkettigen Alkoholen bzw. Alkoholgemischen und den Zuckern bzw. Zucker-liefernden Polymerverbindungen, insbesondere Stärke, oder durch Umacetalisierung primär gewonnener Alkylglykosidverbindungen mit vergleichsweise kurzkettigem Alkoholrest in acetalischer Bindung gewonnen. Die Acetalisierung bzw. Umacetalisierung erfolgt in Gegenwart eines beträchtlichen Überschusses der längerkettigen Alkohole in Gegenwart von sauren Acetalisierungs-Katalysatoren. Zur Reingewinnung der Alkylglykoside aus den Reaktionsrohprodukten bedarf es zunächst der Neutralisation der sauren Katalysatoren und des Abtreibens des Überschusses der langkettigen Alkohole. Diese Alkoholabtrennung erfolgt in der Regel auf dem Wege der Dünnschichtverdampfung bei beträchtlich erhöhten Temperaturen. Sumpftemperaturen im Bereich von 160 bis 210 °C sind hier durchaus üblich. Das anfallende Reaktionsrohprodukt erstarrt im gewonnen. Die Acetalisierung bzw. Umacetalisierung erfolgt in Gegenwart eines beträchtlichen Überschusses der längerkettigen Alkohole in Gegenwart von sauren Acetalisierungs-Katalysatoren. Zur Reingewinnung der Alkylglykoside aus den Reaktionsrohprodukten bedarf es zunächst der Neutralisation der sauren Katalysatoren und des Abtreibens des Überschusses der langkettigen Alkohole. Diese Alkoholabtrennung erfolgt in der Regel auf dem Wege der Dünnschichtverdampfung bei beträchtlich erhöhten Temperaturen. Sumpftemperaturen im Bereich von 160 bis 170 °C sind hier durchaus üblich. Das anfallende Reaktionsrohprodukt erstarrt im Regelfall schon bei Temperaturen oberhalb 100 °C und bildet bei Raumtemperatur fest erstarrte dunkelverfärbte Massen.

Für den praktischen Einsatz der Alkylglykoside beispielsweise als hochwirksame Tensidkomponenten in Wasch- und Reinigungsmitteln für Haushalt und Gewerbe sind diese bis zum Farbton von tief dunkelbraun verfärbten primären Reaktionsprodukte ungeeignet. Regelmäßiger Bestandteil des Syntheseverfahrens ist dementsprechend ein an die Synthese anschließender Arbeitsschritt zur Aufhellung der primär anfallenden Reaktionsrohprodukte. Es besteht ein umfangreicher druckschriftlicher Stand der Technik zur Herstellung oberflächenaktiver Alkylglykoside und ihrer Reingewinnung in Form hellfarbiger Reaktionsprodukte. Verwiesen sei auf die US-PSen 3,450,690 und 3,839,318, die EP 102 558 und 165 721 sowie insbesondere die Deutsche Patentanmeldung P 38 33 780.0 (D 7960 "Verfahren zur direkten Herstellung von Alkylglykosiden") der Anmelderin.

Aus der europäischen Patentanmeldung **EP-A 0 365 831** ist ferner ein Verfahren zur Bleiche von Alkylglykosiden bekannt, zu dessen Durchführung die Tenside jedoch als wäßrige Pasten mit einem Feststoffgehalt von 20 bis 80 Gew.-% vorliegen müssen.

Nach diesen Angaben und insbesondere nach der zuletzt genannten Anmeldung der Anmelderin ist als essentieller Verfahrensschritt des Gesamtverfahrens eine oxidative Bleiche der als primäres Reaktionsrohprodukt in dunklen Farbtönen anfallenden rohen Alkylglykosidverbindungen. Als optimales Bleichmittel haben Aktivsauerstoffverbindung, vorzugsweise Wasserstoffperoxid unter Rühren für einen Zeitraum von ca. 0,1 bis 5 Stunden bei ca. 80 °C der wäßrigen pastenförmigen Zubereitung zugesetzt, wobei man gegebenenfalls durch Hinzufügen von Alkali, vorzugsweise Natronlauge, dafür sorgt, daß der pH-Wert während dieses Bleichprozesses im Bereich von etwa 8 bis 10 gehalten wird. Charakteristisch ist für das bis heute in der Praxis durchgeführte Herstellungsverfahren dieser oxidative Bleichvorgang an der wäßrigen Alkylglykosidpaste, in der das anteilig gelöste Alkylglykoside enthaltende Wasser die geschlossene Phase und der unlösliche Alkylglykosidüberschuß die disperse Phase darstellen. Diese bekannte Verfahrensmaßnahme führt zu hellfarbigen, stabilen Produkten. Die Gewinnung der Alkylglykoside als Trockenstoff oder als Bestandteil trockener Wirkstoffmischungen fordert dann aber stets die Entfernung der beträchtlichen Wassermengen, wodurch das Verfahren kostenmäßig belastet wird.

Die Erfindung geht von der Aufgabe aus, einen Weg aufzuzeigen, auf dem die Herstellung hellfarbiger, insbesondere weißer trockener Alkylglykosidverbindungen unter Einschluß einer Bleiche aber unter Vermeidung der intermediären Bildung der wäßrigen Pastenform möglich ist.

Gegenstand der Erfindung ist ein Verfahren zur Aufhellung verfärbter oberflächenaktiver Alkylglykoside, insbesondere von Reaktions-Rohprodukten aus der Alkylglykosid-Synthese, durch oxidative Bleiche im basischen Bereich und Aufbereitung des gebleichten Gutes, wobei das neue Verfahren dadurch gekennzeichnet ist, daß man das vom Fettalkoholüberschuß weitgehend befreite und höchstens beschränkte Wassermengen enthaltende Material bei derart angehobenen Temperaturen mit dem Oxidationsmittel durchknetet, daß die plastische Verarbeitbarkeit des Reaktionsgemisches sichergestellt ist. Das erfindungsgemäße Verfahren arbeitet insbesondere im Temperaturbereich von etwa 50 bis 150 °C, vorzugsweise im Bereich von etwa 80 bis 100 °C.

Kern der erfindungsgemäßen Lehre ist die neue Erkenntnis, daß es zur wirkungsvollen oxidativen Bleiche der dunkelfarbigen Alkylglykosidverbindungen nicht der bisher als notwendig angesehenen wäßrigen Pastenzubereitung mit geschlossener wäßriger Phase und feinstteiligem festem dispersem Alkylglykosid bedarf, sondern daß unter ausgewählten Reaktionsbedingungen auch das Alkylglykosid selber als homogene Phase des zu bleichenden Gutes wirkungsvoll der Entfärbungsbehandlung unterworfen werden kann.

Entscheidend für dieses Verfahren ist die Eigenschaft der Alkylglykoside, schon vor ihrem Aufschmelzen zur fließfähigen Phase bei erhöhten Temperaturen plastisch derart zu hochviskosen Massen zu erweichen, daß die chemische Reaktion im Sinne der angestrebten Bleiche wirkungsvoll und durch das gesamte plastisch erweichte Gut hindurch vorgenommen werden kann. Es kann zweckmäßig sein, die Produkttemperatur dabei so weit anzuheben, daß plastische, also hochviskose Massen entstehen, die in ihrem Verhalten etwa einem Brotteig, den man in Bäckereien verarbeitet, entsprechen. Die Oxidationsmittel und die Alkalisierungsmittel können in den benötigten geringen Mengen in solche plastifizierten Massen eingemischt und derart homogen darin verteilt werden, daß sowohl die Einstellung des gewünschten pH-Bereiches von wenigstens etwa 8, insbesondere im Bereich von etwa 8 bis 10, als auch die durchgängige Abreaktion des Oxidationsmittels mit den farbgebenden Verunreinigungen sichergestellt sind.

Grundsätzlich können als Oxidationsmittel alle der bekannten Vertreter dieser Art eingesetzt werden, insbesondere also Wasserstoffperoxid, peroxidische Verbindungen etwa von der Art der Perborate, Percarbonsäuren, gewünschtenfalls auch unter jeweiliger Mitverwendung von Bleichaktivatoren, wie sie beispielsweise aus Textilwaschmitteln bekannt sind. Besonders problemlos ist der Einsatz von Wasserstoffperoxid, das letztlich als Reaktionsfolgeprodukt Wasser neben dem gebleichten Produkt liefert, so daß die bevorzugte Ausführungsform des erfindungsgemäßen Verfahrens auf dem Einsatz von Wasserstoffperoxid aufbaut, das durch Mitverwendung eines Alkalisierungsmittels schwach basisch gestellt ist.

Das Oxidationsmittel, insbesondere also das Wasserstoffperoxid, aber auch die zur Einstellung des gewünschten pH-Werts benötigten Alkalisierungsmittel können zusammen mit geringen Mengen an Flüssigphase verwendet werden. Das bevorzugte Lösungsmittel ist hier Wasser, so daß übliche wäßrige Peroxidlösungen bzw. wäßrige Basenlösungen die Zusatzstoffe im Sinne der bevorzugten Ausgestaltung des erfindungsgemäßen Verfahrens sind. Wäßriges Wasserstoffperoxid mit Peroxidkonzentrationen im Bereich von 10 bis 60 Gew.-% und wäßrige Alkalihydroxidlösungen, beispielsweise wäßriges Natriumhydroxid entsprechender Konzentrationsbereiche können besonders geeignete Hilfsmittel im Sinne des erfindungsgemäßen Verfahrens sein. Die wäßrige Lösung oder Dispersion des Oxidationsmittels wird vorzugsweise in einer Menge von unterhalb 10 Gew.-%, insbesondere in einer Menge von 3 bis 8 Gew.-%, eingesetzt. Das Oxidationsmittel und die wäßrige Base werden in der bevorzugten Ausführungsform in so beschränkten Mengen eingesetzt, daß der beschränkte Wassergehalt im plastifizierten Reaktionsgemisch nicht mehr als höchstens etwa 20 Gew.-% ausmacht und vorzugsweise bei 5 bis 10 Gew.-% liegt. Die Einarbeitung dieser Reaktanten bzw. Reaktionshilfsmittel in das plastisch erweichte Alkylglykosid erfolgt insbesondere derart, daß man das plastifizierte Reaktionsgemisch unter permanenter Oberflächenerneuerung intensiv durchknetet. Dabei sind in den angegebenen erhöhten Temperaturbereichen im Bereich um 100 °C Reaktionszeiträume bis zu etwa 15 Minuten völlig ausreichend, um zur Aufhellung oder gar zur vollständigen Entfärbung des rohen Alkylglykosids zu kommen. Ein Behandlungszeitraum unter den erfindungsgemäßen Verfahrensbedingungen bis etwa 10 Minuten und insbesondere ein Bearbeitungszeitraum im Bereich von etwa 3 bis 5 Minuten sind bevorzugte Elemente im Sinne der Erfindung. Die erfindungsgemäßen Verfahrensprodukte eignen sich für solche Einsatzgebiete, in denen gebleichte Alkylglykoside in gebleichter wasserfreier Form benötigt werden. Ein weiterer Vorteil liegt darin, daß die Verfahrensprodukte praktisch keine restlichen Peroxyverbindungen enthalten.

Die Verarbeitung der plastifizierten Alkylglykosidverbindungen und der erfindungsgemäß in geringer Menge eingesetzten Hilfsstoffe der zuvor geschilderten Art kann absatzweise oder kontinuierlich erfolgen. Eine chargenweise Bearbeitung ist beispielsweise in Intensiv-Mischknetern möglich, wie sie beispielsweise zum Kneten von Brotteigen bekannt sind. Bei der großtechnischen Herstellung und Aufarbeitung von Alkylglykosiden wird allerdings die Bleiche im Sinne des erfindungsgemäßen Vorschlages als kontinuierlicher Verfahrensschritt vorgesehen und in das kontinuierlich arbeitende Herstellungsverfahren einbezogen. Möglich ist das in einfacher Form durch den Einsatz von Misch- und Knetextrudern, die erfindungsgemäß als Misch- und Bleichzone eingesetzt und genutzt werden. Es kann dabei zweckmäßig sein, in einer Vorstufe die Plastifizierung der Alkylglykosideinsatzmasse zuerst noch ohne Zusatz von Oxidations- und Alkalisierungsmitteln vorzusehen oder hier nur das Alkalisierungsmittel einzuarbeiten. In einer anschließenden Verfahrensstufe wird dann das wäßrige Wasserstoffperoxid eindosiert, wobei hier auch wieder ein mehrstufiges Eindosieren in aufeinander nachfolgenden Abschnitten des Extruders möglich ist. Durch eine im hinteren Extruderteil vorgesehene Entgasung können gasförmige Zersetzungsprodukte aus der Reaktionsmasse entfernt werden. Am Extruderaustritt tritt das gebleichte und höchstens geringe Wassermengen enthaltende Alkylglykosidprodukt aus. Es kann in verschiedenartigster Weise weiterhin aufgearbeitet werden, wie im nachfolgenden noch geschildert wird.

Das bevorzugte Einsatzmaterial für das erfindungsgemäße Bleichverfahren sind die Reaktionsrohprodukte aus der Alkylglykosidherstellung, so wie sie im heute üblichen Verfahren nach der Abreicherung des Fettalkoholüberschusses anfallen. Charakteristisch für solche Reaktionsrohprodukte sind die Angaben in der genannten Internationalen Patentanmeldung WO90/03977. In dem Beispiel 3 dieser Druckschrift wird der großtechnische Ansatz eines C₁₂/C₁₄-Alkylglukosids beschrieben, wobei nach der Abtrennung des Fettalkoholüberschusses das rohe Alkylglukosid als erstarrte Schmelze anfällt, die ca. 63 Gew.-% Fettalkyl-Monoglukosid, 15 Gew.-% Fettalkyl-Diglukosid, 6 Gew.-% Fettalkyl-Triglukosid, 3 Gew.-% Alkyl-Tetraglukosid, 6 Gew.-% Polyglucose und 2 bis 4 Gew.-% Rest-Fettalkohol enthält. Nach den Angaben dieser Druckschrift wird dieses Produkt chargenweise (90 kg) in geschmolzenem Zustand bei 150 °C in einem Druckkessel mit ca. 88 kg Wasser von Raumtemperatur versetzt, um so eine ca. 50%ige Paste herzustellen. Durch Zudosierung sehr beschränkter Mengen einer 35%igen H₂O₂-Lösung (1,3 kg) und 0,9 kg einer 50%igen NaOH-Lösung wird nach 3stündigem Rühren bei 90 °C der gewünschte Bleicheffekt erzielt.

Die Erfindung verzichtet auf diesen Schritt der Herstellung der wäßrigen Paste. Stattdessen wird die Verfahrensführung im Sinne der erfindungsgemäßen Lehre unmittelbar am plastisch erweichten Material vorgenommen, so wie es im einzelnen den nachfolgenden Beispielen beschrieben ist.

Das gebleichte Reaktionsprodukt, welches den zur Bleiche eingesetzten Extruder verläßt, kann in praktisch beliebiger Form aufgearbeitet werden. Drei charakteristische Möglichkeiten seien hier aufgezählt:
Das den Extruder verlassende Alkylglykosid kann als solches durch Erkaltenlassen und Zerkleinern in eine bestimmte Form eines Feststoffes gebracht werden; beispielsweise wird es zu einem Granulat aufgearbeitet.

Das Alkylglykosid kann andererseits für eine spätere Abmischung mit wäßrigen Flüssigkeiten zur wäßrigen Paste mit beliebigen Feststoffgehalten, beispielsweise solchen im Bereich von etwa 40 bis 60 Gew.-%, aufgearbeitet werden.

In der dritten Ausführungsform, die für viele Anwendungszwecke besondere Bedeutung haben wird, kann das gereinigte Alkylglykosid mit festen Stoffen vereinigt, insbesondere mit diesen intensiv vermengt werden. Die anfallenden Stoffgemische können dann wiederum beispielsweise der Granulation zugeführt werden.

In der zuletzt dargestellten Aufarbeitung sind zahlreiche Ausführungsformen im Sinne des erfindungsgemäßen Handelns erfaßt. Die den Alkylglykosiden zugesetzten Stoffe sind Hilfsstoffe im weitesten Sinne, die Eigenwirkung in dem nachfolgenden Einsatzzweck der Alkylglykoside besitzen oder aber auch nur als Hilfsstoffe für die verbesserte oder vereinfachte technische Verwertung der Alkylglykoside anzusehen sind. Beispielhafte Angaben für mögliche Zusatzstoffe finden sich in der älteren Internationalen Patentanmeldung WO91/02046 ("Pulverförmige Zubereitungen oberflächenaktiver Alkylglykoside") des Anmelders. Beschrieben sind hier pulverförmige Zubereitungen oberflächenaktiver Alkylglykoside in Abmischung mit inerten anorganischen Trägern, wobei die Alkylglykoside 5 bis 65 Gew.-% und die inerten Trägerstoffe den Rest des Stoffgemisches ausmachen. Als anorganische Träger sind beispielsweise genannt Kreide, Kieselsäuren, Dicalciumphosphat, Calciumpyrophosphat, wasserunlösliches Natriummetaphosphat, Aluminiumoxid, Aluminiumoxid-hydrat, Kochsalz, Natriumsulfat oder Gemische aus diesen Stoffen. Stoffmischungen dieser Art sind insbesondere für den Einsatz auf dem Kosmetikgebiet, beispielsweise in Mund- und Zahnpflegemitteln gedacht.

Ganz andere interessante Mischungskomponenten fallen in den Bereich der Erfindung, wenn an andere charakteristische Einsatzzwecke der Alkylglykoside gedacht wird. Hier handelt es sich insbesondere um das Gebiet der Wasch- und/oder Reinigungsmittel für Haushalt und Gewerbe. Die Alkylglykoside können hier mit typischen, bevorzugt feinteiligen Inhaltsstoffen solcher Wasch- und Reinigungsmittel abgemischt werden und mit ihnen zusammen dann zum Granulat aufgearbeitet werden. Lediglich beispielhaft seien Alkalisierungsmittel wie Soda oder Builderkomponenten wie Alkalipolyphosphate und insbesondere Zeolith NaA in Waschmittelqualität genannt. Ebenso können aber auch bereits in diesem Stadium Abmischungen der Alkylglykoside mit weiteren tenisidischen Komponenten in Betracht kommen. Es ist dabei möglich, das Alkylglykosid in praktisch beliebigem Mengenverhältnis in das entstehende Mehrstoffgemisch einzuarbeiten, so daß zahlenmäßig der Bereich von 5 bis 95 Gew.-% Alkylglykosid auf 95 bis 5 Gew.-% des oder der Mischungskomponente(n) in Betracht kommen.

In dem zuletzt angegebenen Zusammenhang bestehen interessante Möglichkeiten für die Zubereitung der Alkylglykosid enthaltenden Mehrstoffgemische. Beispielhaft sei das an den folgenden Überlegungen dargestellt:

Werden als Mischungskomponenten zusammen mit den gebleichten Alkylglykosiden wenigstens anteilig solche Feststoffe eingesetzt, die durch Wasserbindung unter interner Kristallwasserbildung in der Lage sind, im Mehrstoffgemisch vorliegendes Wasser zu immobilisieren, so kann ein Mehrgutkorn hergestellt werden, das von substantiellen Anteilen des bei der Bleiche eingebrachten Wassers befreit oder gar vollständig aufgetrocknet ist. Ein klassisches Beispiel hierfür ist die Abmischung mit calciniertem Soda oder Natriumsulfat in wasserarmer bzw. wasserfreier Form.

Auf der anderen Seite kann durch Mitverwendung von Hilfsstoffen der rasche Zerfall eines solchen intermediär gebildeten Mehrstoffkornes gefördert werden. Geeignet sind hier insbesondere sogenannte Sprengmittel und/oder kaltlösliche Hilfsstoffe. Typische Beispiele für Sprengmittel sind anorganische Komponenten, beispielsweise von der Art der quellfähigen Schichtsilikate wie Bentonite oder organische entsprechende Materialien auf Naturstoffbasis bzw. auf Basis von Naturstoffderivaten. Zu nennen sind hier insbesondere Stärke, Cellulose und/oder deren Derivate wie CMC, MC, Alginatverbindungen und dergleichen. Kaltlösliche Hilfsstoffe sind leicht lösliche Salze, beispielsweise Natriumacetat, Harnstoff aber auch Percarbonate bzw. beliebige andere entsprechende kaltlösliche Hilfsstoffe mit oder ohne Eigenwirkung für den beabsichtigten späteren Anwendungszweck, beispielsweise in Wasch- und Reinigungsmitteln. Besondere Bedeutung können in diesem Zusammenhang auch rein synthetische Hilfsstoffe haben, wie sie beispielsweise in Form der Alkalisalze von Polyacrylaten bzw. Polymethacrylaten vergleichsweise niederen Molekulargewichts bekannt sind. Polymerkomponenten dieser Art mit durchschnittlichen Molgewichten von etwa 1000 bis 5000 und insbesondere im Bereich von etwa 1000 bis 3000 zeichnen sich durch starke Dispergierwirkung schon bei sehr geringem Einsatz aus, so daß hier schon Zusatzmengen unter 10 Gew. -%, vorzugsweise unter 1 Gew.-% zu einer substantiellen Beschleunigung des Primärzerfalls der Granulatkörner führen können.

### BEISPIELE

### Beispiel 1

Einem Dünnschichtverdampfer wurde die Menge von 1 kg Alkylglucosid (C₁₂/C₁₄-Alkyl, Oligomerisierungsgrad 1,4) unmittelbar nach der Fettalkohol-Abreicherung auf 2 bis 3 Gew.-% entnommen und in einen beheizbaren Labor-Kneter gegeben (Temperatur der Schmelze, ca. 120 °C). Man ließ auf ca. 100 °C abkühlen und gab dann 40 g einer 50%igen Natriumhydroxid-Lösung (2 Gew.-% Natriumhydroxid, bezogen auf das Alkylglucosid) unter Vermischen hinzu. Man erhielt so eine plastische Masse von 80 °C Temperatur, zu der man unter ständigem Kneten 67 g einer 30%igen wäßrigen H₂O₂-Lösung innerhalb von 30 Minuten hinzugab. Anschließend wurde noch bei 80 °C 15 Minuten lang durchgemischt. Danach wurde die plastische Masse ausgetragen und nach dem Abkühlen unter Zerkleinern mit 1 kg Natriumcarbonat-Pulver versetzt und zu einem weißen rieselfähigen Granulat homogenisiert.

### Beispiel 2

Es wurden 5 kg des Alkylglucosids des Beispiel 1 in einem beheizbaren Labor-Kneter bei 90 °C mit den wäßrigen Natriumhydroxid- und Wasserstoffperoxid-Lösungen vermischt, wobei man zunächst 100 g einer 50%igen Natriumhydroxid-Lösung hinzumischte und dabei eine Temperatur von etwa 90 °C einstellte und dann 168 g einer 30%igen H₂O₂-Lösung innerhalb von 30 Minuten hinzugab. Nachmischzeit bei 90 °C: 15 Minuten. Es wurde ein schwach gelbes Reaktionsprodukt erhalten, das nach dem Zerkleinern und Homogenisieren mit 10 kg Zeolith A zu einem weißen rieselfähigen Granulat vermischt wurde.

Bei der Durchführung des erfindungsgemäßen Verfahrens in einem Extruder als kontinuierlich arbeitender Knetvorrichtung ließen sich die Bearbeitungszeiten gegenüber denen im Laborkneter auf Werte von unter 15 Minuten kürzen.

### Beispiel 3

150 g eines C₈-Alkylpolyglucosides (Oligomerisierungsgrad 1,7) wurden in einem Laborreaktor bei 90 °C Innentemperatur mit 1,5 g NaOH (50%ig) und anschließend mit 2,5 g H₂O₂ (30%ig) versetzt. 30 Minuten später wurde die Temperatur auf 110 °C erhöht und für zwei Stunden gehalten. Während der letzten 30 Minuten wurde Vakuum (ca. 1 - 10 mbar) zur Entfernung des Wassers angelegt. Dann wurden bei ca. 100 °C 75 g Na₂SO₄ (wasserfrei) hinzugegeben. Nach einer gründlichen Durchmischung wurde das erhaltene Gemisch bei Raumtemperatur gemahlen (Labormixer). Die Farbqualität wurde als Klett-Zahl (3%ig in Isopropanol/Wasser) mit 11 ermittelt. Die Peroxid-Zahl wurde mit dem Wert kleiner als 1 ermittelt.

### Beispiel 4

150 g eines C_{12/14}-Alkylpolyglucosides (Oligomerisierungsgrad 1,4) wurden in einem Laborkneter bei 90 °C Innentemperatur mit 3,0 g NaOH (50%ig) und anschließend mit 5,0 g H₂O₂ (30%ig) versetzt. 30 Minuten später wurde die Temperatur auf 110 °C erhöht und für zwei Stunden gehalten. Während der letzten 30 Minuten wurde Vakuum (ca. 1 - 10 mbar) zur Entfernung des Wassers angelegt. Das Produkt wird bei Raumtemperatur gemahlen (Labormixer). Klett-Zahl (5%ig in Isopropanol/Wasser): 17. Peroxid-Zahl: kleiner als 1.

### Beispiel 5

150 g eines C₁₆-Alkylpolyglucosides (Oligomerisierungsgrad 1,4) wurden in wie in Beispiel 4 mit 1,5 g NaOH (50%ig) und 2,5 g H₂O₂ (30%ig) behandelt. Es wurde so ein sehr hellfarbiges Pulver mit der Klett-Zahl (5%ig in Toluol) von 25 erhalten. Peroxid-Zahl kleiner als 1.

## Patentansprüche

1. Verfahren zur Aufhellung verfärbter oberflächenaktiver Alkylglykoside, insbesondere von Reaktionsrohprodukten aus der Alkylglykosid-Synthese, durch oxidative Bleiche im basischen Bereich und Aufbereitung des gebleichten Gutes, dadurch gekennzeichnet, daß man das vom Fettalkoholüberschuß weitgehend befreite und höchstens beschränkte Wassermengen enthaltende Material bei derart angehobenen Temperaturen mit dem Oxidationsmittel durchknetet, daß die plastische Verarbeitbarkeit des Reaktionsgemisches sichergestellt ist.

2. Verfahren nach Anspruch 1, dadurch gekennzeichnet, daß man bei Temperaturen von 50 bis 150 °C, vorzugsweise im Bereich von etwa 80 bis 100 °C arbeitet.

3. Verfahren nach Ansprüchen 1 und 2, dadurch gekennzeichnet, daß man als Oxidationsmittel schwach basisch gestelltes wäßriges Wasserstoffperoxid einsetzt, wobei bevorzugt mit einem Wassergehalt im plastifizierten Reaktionsgemisch nicht über 20 Gew.-%, vorzugsweise bei 5 bis 10 Gew. -%, gearbeitet wird.

4. Verfahren nach Ansprüchen 1 bis 3, dadurch gekennzeichnet, daß das plastifizierte Reaktionsgemisch unter permanenter Oberflächenerneuerung intensiv durchgeknetet wird, wobei Bearbeitungszeiten nicht über 15 Minuten, vorzugsweise im Bereich von etwa 3 bis 5 Minuten bevorzugt sind.

5. Verfahren nach Ansprüchen 1 bis 4, dadurch gekennzeichnet, daß chargenweise in Intensiv-Mischknetern, vorzugsweise aber im kontinuierlichen Verfahren im Extruder als Misch- und Bleichzone gearbeitet wird, wobei zweckmäßigerweise Peroxid und Alkalisierungsmittel absatzweise oder kontinuierlich unmittelbar in den Extruder eingespeist werden.

6. Verfahren nach Ansprüchen 1 bis 5, dadurch gekennzeichnet, daß das gebleichte Alkylglykosid-Produkt als solches in die Form eines Feststoffes gebracht, z. B. granuliert wird, vorzugsweise aber mit feinteiligen Feststoffen vermischt und gewünschtenfalls zu granuliertem Gut verarbeitet oder mit einer flüssigen Trägerphase, insbesondere Wasser, zur pastenförmigen Konsistenz aufgearbeitet wird.

7. Verfahren nach Ansprüchen 1 bis 6, dadurch gekennzeichnet, daß man die gebleichten Alkylglykoside mit feinteiligen löslichen und/oder unlöslichen Hilfsstoffen, insbesondere Inhaltsstoffen von Wasch- und/oder Reinigungsmitteln verknetet und zu Formkörpern, insbesondere Granulaten aufarbeitet.

## Claims

1. A process for bleaching discolored surface-active alkyl glycosides, more particularly crude reaction products from the synthesis of alkyl glycosides, by oxidative bleaching in the basic range and working up of the bleached material, characterized in that the material substantially freed from the excess fatty alcohol and containing at most limited quantities of water is kneaded with the oxidizing agent at such elevated temperatures that the reaction mixture lends itself to plastic processing.

2. A process as claimed in claim 1, characterized in that it is carried out at temperatures of 50 to 150°C and preferably at temperatures from about 80 to 100°C.

3. A process as claimed in claims 1 and 2, characterized in that mildly alkalized aqueous hydrogen peroxide is used as the oxidizing agent, the water content of the plasticized reaction mixture being no more than 20% by weight and preferably between 5 and 10% by weight.

4. A process as claimed in claims 1 to 3, characterized in that the plasticized reaction mixture is intensively kneaded with permanent surface renewal, the kneading times being no more than 15 minutes and preferably between about 3 and 5 minutes.

5. A process as claimed in claims 1 to 4, characterized in that it is carried out in batches in intensive kneaders and, preferably, continuously in an extruder as the mixing and bleaching zone, peroxide and alkalizing agent best being directly introduced into the extruder either in batches or continuously.

6. A process as claimed in claims 1 to 5, characterized in that the bleached alkyl glycoside product is brought as such into the form of a solid, for example granulated, but is preferably mixed with fine-particle solids and, if desired, processed to granules or worked up with a liquid carrier phase, particularly water, to a paste-like consistency.

7. A process as claimed in claims 1 to 6, characterized in that the bleached alkyl glycosides are kneaded with fine-particle soluble and/or insoluble auxiliaries, more particularly ingredients of detergents and/or cleaning preparations, and worked up to shaped solids, more particularly granules.

## Revendications

1. Procédé pour éclaircir des alkylglycosides tensioactifs décolorés, plus particulièrement des produits bruts de réaction issus de la synthèse des alkylglycosides, par blanchiment par oxydation dans le domaine basique, et pour traiter le produit blanchi, caractérisé en ce que l'on pétrit intimement avec l'oxydant le matériel largement débarrassé de l'excès d'alcool gras et contenant des quantités d'eau extrêmement limitées, à des températures accrues au point que la transformabilité plastique du mélange réactionnel est garantie.

2. Procédé selon la revendication 1, caractérisé en ce que l'on travaille à des températures de 50 à 150 °C, de préférence comprises dans l'intervalle d'environ 80 à 100 °C.

3. Procédé selon les revendications 1 et 2, caractérisé en ce que l'on utilise comme oxydant du peroxyde d'hydrogène aqueux rendu légèrement basique, en travaillant de préférence avec une concentration en eau dans le mélange réactionnel plastifié ne dépassant pas 20 % en poids, de préférence comprise entre 5 et 10 % en poids.

4. Procédé selon les revendications 1 à 3, caractérisé en ce que le mélange réactionnel plastifié est pétri intimement de manière intensive, sous renouvellement permanent de la surface, les durées de traitement n'étant pas supérieures à 15 minutes, de préférence comprises dans l'intervalle d'environ 3 à 5 minutes.

5. Procédé selon les revendications 1 à 4, caractérisé en ce que l'on travaille de manière discontinue dans des malaxeurs-mélangeurs intensifs, de préférence toutefois en procédé continu dans une extrudeuse comme zone de mélangeage et de blanchiment, le peroxyde et l'agent d'alcalinisation étant de préférence alimentés directement dans l'extrudeuse de manière discontinue ou continue.

6. Procédé selon les revendications 1 à 5, caractérisé en ce que le produit d'alkylglycoside blanchi est transformé en tant que tel sous la forme d'une' matière solide, par exemple granulé, mais de préférence mélangé avec des matières solides à fines particules et éventuellement transformé en produit granulé, ou traité par une phase porteuse liquide, en particulier de l'eau, pour prendre une consistance pâteuse.

7. Procédé selon les revendications 1 à 6, caractérisé en ce que l'on pétrit les alkylglycosides blanchis avec des adjuvants à fines particules solubles et/ou insolubles, en particulier des constituants d'agents de lavage et/ou de nettoyage, et en ce qu'on les transforme en pièces moulées, en particulier, en granulés.
